# EUROPEAN PATENT SPECIFICATION

(11) **EP 3 773 177 B1**
(45) Date of publication and mention of the grant of the patent: **07.08.2024**
(21) Application number: 19822720.9
(22) Date of filing: 04.06.2019
(51) Int. Cl.: A61B 5/00, A61B 5/145, A61B 5/1455, A61B 5/021

(54) **BIOMETRIC INFORMATION SENSING DEVICE**
BIOMETRISCHE INFORMATIONSERFASSUNGSVORRICHTUNG
DISPOSITIF DE DÉTECTION D'INFORMATIONS BIOMÉTRIQUES

(30) Priority: 19.06.2018 KR 20180070131
(43) Date of publication of application: 17.02.2021
(73) Proprietor: Samsung Electronics Co., Ltd., Gyeonggi-do 16677 (KR)
(72) Inventor: PARK, Jaehyuck, Suwon-si, Gyeonggi-do 16677 (KR); CHO, Minhyun, Suwon-si, Gyeonggi-do 16677 (KR); KWON, Hyoujoo, Suwon-si, Gyeonggi-do 16677 (KR); KIM, Jungmo, Suwon-si, Gyeonggi-do 16677 (KR); KIM, Taehyeon, Suwon-si, Gyeonggi-do 16677 (KR); WON, Jinhee, Suwon-si, Gyeonggi-do 16677 (KR); LEE, Wonseok, Suwon-si, Gyeonggi-do 16677 (KR); LIM, Daehyeong, Suwon-si, Gyeonggi-do 16677 (KR); JEONG, Daeung, Suwon-si, Gyeonggi-do 16677 (KR)
(74) Representative: Gulde & Partner
(86) International application number: PCT/KR2019/006729
(87) International publication number: WO 2019/245197

(56) References cited:
- JP-A- 2016 086 873
- JP-A- 2016 195 853
- JP-A- 2017 148 584
- KR-A- 20170 004 607
- US-A1- 2013 060 098
- US-A1- 2016 206 251
- US-A1- 2017 000 350
- US-A1- 2017 156 651
- US-A1- 2017 315 511

## Description

### [Technical Field]

The disclosure relates to a biometric information sensing device.

### [Background Art]

As various portable electronic devices such as smart phones are distributed, a method of sensing biometric information using a portable electronic device is being studied. A health care service may be provided to a user, by sensing the biometric information using the portable electronic device. Methods of sensing accurate biometric information are being studied to provide an accurate health care service. At the same time, methods of providing complex health care services using various pieces of biometric information are being studied.

US 2017/000350 A1 discloses an apparatus for detecting biological information comprising multiple LEDs arranged in a matrix and surrounded by multiple photodetectors, wherein the LEDs have different light emission angles to obtain a high SNR regardless of the distance between the photodetectors and a measurement surface of a subject.

The above information is presented as background information only to assist with an understanding of the disclosure. No determination has been made, and no assertion is made, as to whether any of the above might be applicable as prior art with regard to the disclosure.

### [Disclosure]

### [Technical Problem]

A portable electronic device may be configured to detect various pieces of biometric information to provide a complex health care service. Because the portable electronic device has a small size, all various sensors for sensing biometric information may fail to be mounted in the portable electronic device. Accordingly, a sensor that requires a small mounting space and is capable of sensing various pieces of biometric information may be used in portable electronic devices.

Aspects of the disclosure are to address at least the above-mentioned problems and/or disadvantages and to provide at least the advantages described below. Accordingly, an aspect of the disclosure is to provide a biometric information sensor capable of obtaining various pieces of biometric information and an electronic device including the same. Aspects of the disclosure are to provide a structure of a biometric information sensor configured to obtain various pieces of biometric information.

### [Technical Solution]

The invention is defined by the appended claims. The description that follows is subjected to this limitation. Any disclosure lying outside the scope of said claims is only intended for illustrative as well as comparative purposes.

### [Advantageous Effects]

According to various embodiments disclosed in the disclosure, accurate various pieces of biometric information may be detected.

Besides, a variety of effects directly or indirectly understood through this disclosure may be provided.

Other aspects, advantages, and salient features of the disclosure will become apparent to those skilled in the art from the following detailed description, which, taken in conjunction with the annexed drawings, discloses various embodiments of the disclosure.

### [Description of Drawings]

The above and other aspects, features, and advantages of certain embodiments of the disclosure will be more apparent from the following description taken in conjunction with the accompanying drawings, in which:
FIG. 1 illustrates a block diagram of the electronic device in a network, according to various embodiments of the disclosure;
FIG. 2 illustrates a block diagram of an electronic device and a biometric information sensor, according to various embodiments of the disclosure;
FIG. 3 is a configuration diagram of a biometric information sensor, according to an embodiment of the disclosure;
FIG. 4 illustrates an arrangement of a biometric information sensor, according to various embodiments of the disclosure;
FIG. 5a illustrates a first example of a biometric information sensor, according to various embodiments of the disclosure;
FIG. 5b illustrates a second example of a biometric information sensor, according to various embodiments of the disclosure;
FIG. 6 illustrates a configuration of an electronic device, according to various embodiments of the disclosure;
FIG. 7 illustrates a mounting structure of a biometric information sensor of an electronic device, according to various embodiments of the disclosure;
FIG. 8 illustrates a configuration on a printed circuit board (PCB) of a biometric information sensor, according to various embodiments of the disclosure;
FIG. 9 illustrates another configuration of an electronic device, according to various embodiments of the disclosure;
FIG. 10 illustrates a biometric information sensor in an in-display form, according to various embodiments of the disclosure;
FIG. 11 illustrates an exemplification of a structure of a pixel, according to various embodiments of the disclosure;
FIG. 12 illustrates another exemplification of a structure of a pixel, according to various embodiments of the disclosure;
FIG. 13 illustrates still another exemplification of a structure of a pixel, according to various embodiments of the disclosure;
FIG. 14 illustrates a layer structure for sensing biometric information, according to various embodiments of the disclosure; and
FIG. 15 illustrates a biometric information management environment, according to various embodiments of the disclosure.

Throughout the drawings, like reference numerals will be understood to refer to like parts, components, and structures.

### [Mode for Invention]

The following description with reference to the accompanying drawings is provided to assist in a comprehensive understanding of various embodiments of the disclosure as defined by the claims. It includes various specific details to assist in that understanding but these are to be regarded as merely exemplary. Accordingly, those of ordinary skill in the art will recognize that various changes and modifications of the various embodiments described herein can be made without departing from the scope of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims.

It is to be understood that the singular forms "a," "an," and "the" include plural referents unless the context clearly dictates otherwise. Thus, for example, reference to "a component surface" includes reference to one or more of such surfaces.

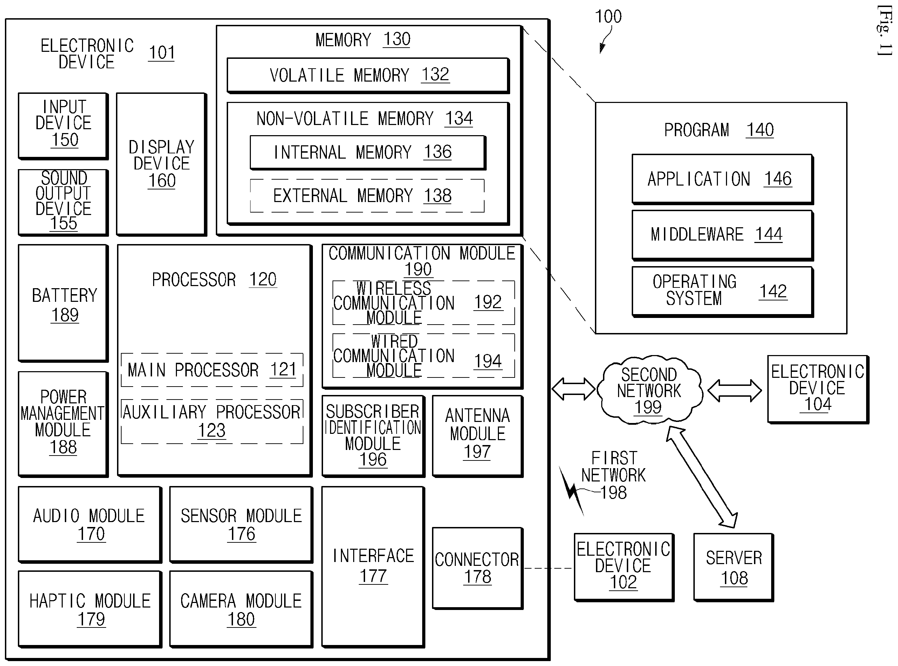

FIG. 1 is a block diagram illustrating an electronic device in a network environment according to various embodiments of the disclosure.

Referring to FIG. 1, an electronic device in a network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module (SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display device 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an ISP or a CP) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, or a keyboard.

The sound output device 155 may output sound signals to the outside of the electronic device 101. The sound output device 155 may include, for example, a speaker or a receiver. The speaker may be used for general purposes, such as playing multimedia or playing record, and the receiver may be used for incoming calls. According to an embodiment, the receiver may be implemented as separate from, or as part of the speaker.

The display device 160 may visually provide information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain the sound via the input device 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector),

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, ISPs, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more CPs that are operable independently from the processor 120 (e.g., the AP) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The antenna module 197 may transmit or receive a signal or power to or from the outside (e.g., the external electronic device) of the electronic device 101. According to an embodiment, the antenna module 197 may include one or more antennas, and, therefrom, at least one antenna appropriate for a communication scheme used in the communication network, such as the first network 198 or the second network 199, may be selected, for example, by the communication module 190 (e.g., the wireless communication module 192). The signal or the power may then be transmitted or received between the communication module 190 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 and 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

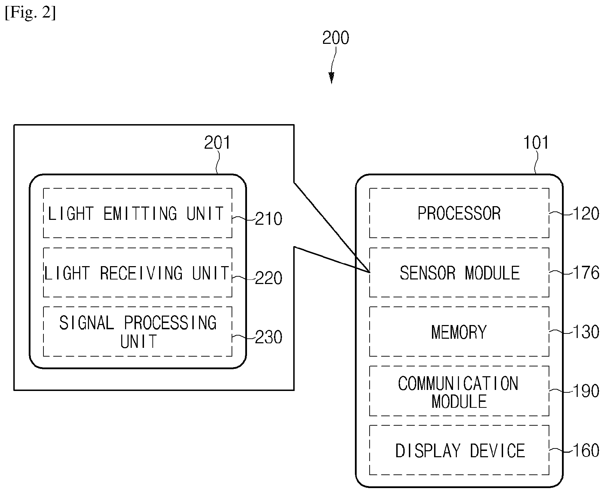

FIG. 2 illustrates a block diagram of an electronic device and a biometric information sensor, according to various embodiments of the disclosure.

Referring to FIG. 2, an apparatus 200 may include the electronic device 101, which may include at least one of the processor 120, the sensor module 176, the memory 130, the communication module 190, or the display device 160. Hereinafter, unless otherwise described, the description of a configuration having the same identification number may be referred to by the above-described details with reference to FIG. 1. Thus, additional description will be omitted to avoid redundancy. The configuration of the electronic device 101 illustrated in FIG. 2 is an aspect, and the electronic device 101 may not include a part of the components illustrated in FIG. 2 or may further include components not illustrated in FIG. 2.

According to various embodiments, the processor 120 may be electrically or operatively coupled to other components (e.g., at least one of the sensor module 176, the memory 130, the communication module 190, or the display device 160) of the electronic device 101 and may be configured to control other components of the electronic device 101. In the following embodiments, the operation of the electronic device 101 may be referred to as the operation of the processor 120.

According to various embodiments, the memory 130 may be electrically connected to the processor 120 and may store at least one instruction that causes the processor 120 to perform various operations. In the following embodiments, the operation of the processor 120 may be performed based on instructions stored in the memory 130.

According to various embodiments, the display device 160 may include a display. According to an embodiment, the display may provide a user with visual information and may be configured to receive a user input (e.g., a touch input). According to an embodiment, an indicator may be configured to generate light having at least one wavelength through a front side (e.g., a surface on which the display of the electronic device 101 is positioned) of a housing of the electronic device 101. According to an embodiment, the display device 160 may include a plurality of pixels and at least part of the plurality of pixels may be used as a light emitting unit 210 of the sensor module 176.

According to various embodiments, the communication module 190 may be configured to communicate with an external electronic device (e.g., the electronic device 102, the electronic device 104, or the server 108 of FIG. 1) over various networks (e.g., the second network 199 and/or a first network 198 of FIG. 1). According to an embodiment, the processor 120 may transmit information associated with the electronic device 101 to the external electronic device, using the communication module 190. For example, the electronic device 101 may transmit, to the external electronic device, data sensed by a biometric information sensor 201 or related data obtained based on the sensed data, using the communication module 190.

According to various embodiments, the sensor module 176 may include at least one sensor for sensing information associated with a surrounding environment of the electronic device 101 and/or an external object (e.g., a user and the like). According to an embodiment, the sensor module 176 may include at least one of a proximity sensor, the biometric information sensor 201, or a motion sensor. For example, the proximity sensor may be positioned on the front side of the housing of the electronic device 101 and may sense whether there is an external object positioned within a specified range from the proximity sensor. For example, the motion sensor may be positioned on the inside of the housing of the electronic device 101 and may include at least one sensor (a gyro sensor and/or an acceleration sensor) for sensing the posture, tilt, movement, and the like of the electronic device 101.

According to various embodiments, the biometric information sensor 201 may include the light emitting unit 210, a light receiving unit 220, and/or a signal processing unit 230. According to an embodiment, the processor 120 may detect biometric information (e.g., heart rate, oxygen saturation, blood pressure, and/or blood glucose) associated with an external object (e.g., a user or the like), using the biometric information sensor 201.

According to various embodiments, the light emitting unit 210 may include at least one light emitting element (e.g., a light emitting diode (LED)) for emitting light of a wavelength of a specified range. According to an embodiment, the light emitting unit 210 may include at least one light emitting element for emitting light having a wavelength (e.g., about 400 nm ~ about 1000 *µ*m) of a specified range. According to an embodiment, the light emitting unit 210 may include a plurality of light emitting elements for emitting light corresponding to a plurality of wavelengths. For example, the light emitting unit 210 may include a plurality of light emitting elements respectively corresponding to red (wavelength: about 600 nm ~ 700 nm), green (wavelength: about 500 ~ 600 nm), blue (wavelength: about 400 nm ~ 500 nm), and/or infrared light (wavelength: about 780 nm ~ 1000 ,um). According to an embodiment, the light emitting unit 210 may be implemented on the display device 160. For example, at least part of the light emitting unit 210 may be implemented using a pixel or a sub-pixel of the display device 160.

According to various embodiments, the light receiving unit 220 may include at least one light receiving element for detecting light. According to an embodiment, the light receiving element may detect the light of a specified wavelength and may detect the intensity of the detected light. For example, the light receiving element may output a current signal of a magnitude corresponding to the intensity of the detected light. For example, the light receiving element may include a photodetector or a photo diode. The photo diode is an example of a photodetector, and the light receiving element of the disclosure may be an arbitrary element capable detecting light. According to an embodiment, the light receiving unit 220 may include at least one photodetector (e.g., a photo diode) for detecting light having a wavelength (e.g., about 400 nm ~ about 1000 *µ*m) of a specified range. According to an embodiment, the light receiving unit 220 may include a plurality of light receiving elements for detecting light corresponding to a plurality of wavelengths. For example, the light receiving unit 220 may include a plurality of photodetectors configured to detect light corresponding to red, green, blue, and/or infrared, respectively.

According to various embodiments, the signal processing unit 230 may control the light emitting unit 210 and the light receiving unit 220. For example, the signal processing unit 230 may control the light emitting unit 210 and/or the light receiving unit 220, under the control of the processor 120. According to an embodiment, the signal processing unit 230 may drive at least one LED of the light emitting unit 210. According to an embodiment, the signal processing unit 230 may process a signal detected by the light receiving unit 220. For example, the signal processing unit 230 may convert the current signal detected by the light receiving unit 220 to a voltage signal, may process (e.g., amplify and/or filter) the voltage signal, and may convert the processed voltage signal to a digital signal. According to an embodiment, the signal processing unit 230 may include a memory for storing biometric information detected by the light receiving unit 220 and/or instructions for controlling the light receiving unit 220 and the light emitting unit 210. According to an embodiment, the processor 120 may perform post-processing (e.g., filtering and/or noise cancelling) on the biometric information detected by the biometric information sensor 201.

According to various embodiments, the biometric information sensor 201 may emit light via one surface (e.g., rear side or front side) of the electronic device 101 and may receive (e.g., sense or detect) the light reflected from an external object (e.g., a user). For example, the biometric information sensor 201 may sense light or emit light, via a window having translucency formed in at least part of the housing of the electronic device 101.

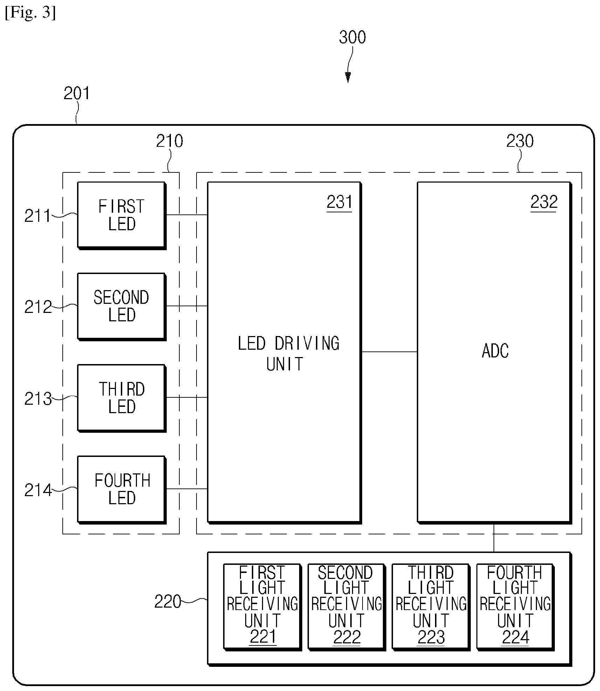

FIG. 3 is a configuration diagram of a biometric information sensor, according to an embodiment of the disclosure.

Referring to FIG. 3, according to various embodiments, an apparatus 200 may include the light emitting unit 210 which may include at least one of a first LED 211, a second LED 212, a third LED 213, or a fourth LED 214. The number of LEDs included in the light emitting unit 210 is an aspect for description, and the number of LEDs is not limited thereto. According to an embodiment, the first LED 211 may be a green LED; the second LED 212 may be a blue LED; the third LED 213 may be a red LED; the fourth LED 214 may be an infrared LED. According to an embodiment, the light emitting unit 210 may include at least one LED capable of emitting a wavelength corresponding to at least one of blue light, green light, red light, and infrared light. For example, the light emitting unit 210 may include a single LED capable of simultaneously emitting all wavelengths corresponding to blue light, green light, red light, and infrared light.

According to various embodiments, a signal processing unit 230 may include an LED driving unit 231 and an analog to digital converter (ADC) 232. The signal processing unit 230 may further include other components (e.g., amplifiers, filters, and/or memories) not illustrated in FIG. 3.

According to various embodiments, the LED driving unit 231 may control the light emitting unit 210 (e.g., at least one of the first LED 211, the second LED 212, the third LED 213, or the fourth LED 214) in a specified state. For example, a processor (e.g., the processor 120 of FIG. 2) may control the light emitting unit 210, using the LED driving unit 231. According to an embodiment, the LED driving unit 231 may drive the light emitting unit 210 in an always-on state. According to an embodiment, the LED driving unit 231 may turn on the light emitting unit 210 during a specified time (e.g., about 1 ,us ~ about 10 ms). According to an embodiment, the LED driving unit 231 may flicker the light emitting unit 210 at a specified period. For example, the LED driving unit 231 may control the light emitting unit 210 in a sensed state (e.g., flicking at a sensor sample rate (e.g., about 10 - 1000 Hz)).

According to various embodiments, the ADC 232 may convert the analog signal detected by the light receiving unit 220, to a digital signal. According to various embodiments, the light receiving unit 220 may include a plurality of light receiving elements (e.g., photodetectors). According to an embodiment, the light receiving unit 220 may include a first light receiving unit 221, a second light receiving unit 222, a third light receiving unit 223, and/or a fourth light receiving unit 224. The four light receiving units are an aspect, and the light receiving unit 220 may include a plurality of light receiving units.

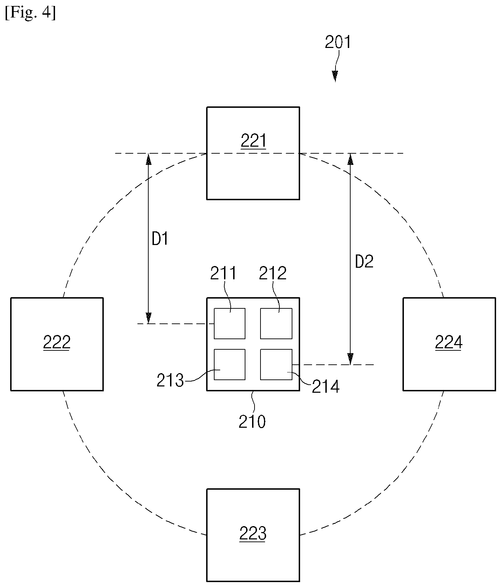

FIG. 4 illustrates an arrangement of a biometric information sensor, according to various embodiments of the disclosure.

Referring to FIG. 4, according to various embodiments, the light emitting unit 210 of a biometric information sensor (e.g., the biometric information sensor 201 of FIG. 2) may include a plurality of light emitting elements. According to an embodiment, the light emitting unit 210 may include the first LED 211, the second LED 212, the third LED 213, and the fourth LED 214. According to an embodiment, the first LED 211, the second LED 212, the third LED 213, and the fourth LED 214 may be positioned in the light emitting unit 210 in a matrix form. Each of the first LED 211, the second LED 212, the third LED 213, and the fourth LED 214 may be positioned at equidistance from the center of the light emitting unit 210. Four LEDs are an aspect, and the biometric information sensor 201 may include LEDs of the first number (e.g., an integer greater than or equal two).

According to various embodiments, a plurality of LEDs 211, 212, 213, and 214 of the light emitting unit 210 may be configured to emit light of different wavelengths. For example, each of the plurality of LEDs 211, 212, 213, and 214 may be configured to emit light with different penetration depths with respect to the body tissue. For example, the first LED 211 may be configured to emit green light; the second LED 212 may be configured to emit blue light; the third LED 213 may be configured to emit infrared light; the fourth LED 214 may be configured to emit red light.

According to various embodiments, the biometric information sensor 201 may control light strength of each of the plurality of LEDs 211, 212, 213, and 214 of the light emitting unit 210. According to an embodiment, the biometric information sensor 201 may generate the light of a wavelength of a wide band, using a plurality of LEDs of the light emitting unit 210. For example, the biometric information sensor 201 may generate the light of a wavelength of a wide band, by adjusting the turn-on/turn-off and light intensity of a plurality of LEDs of the light emitting unit 210. For example, the biometric information sensor 201 may generate the light of a wavelength of a wide band from a plurality of LEDs, based on the spectroscopy of the light emitting unit 210.

According to various embodiments, the biometric information sensor 201 may include a plurality of light receiving elements (e.g., photodetectors). According to an embodiment, the biometric information sensor 201 may include the first light receiving unit 221, the second light receiving unit 222, the third light receiving unit 223, and the fourth light receiving unit 224. Four light receiving units are an aspect, and the biometric information sensor 201 may include LEDs of the second number (e.g., an integer greater than or equal to two). According to an embodiment, a plurality of light receiving elements may be positioned at equidistance from the light emitting unit 210. For example, a plurality of light receiving elements may be positioned at equidistance from the center of the light emitting unit 210. According to an embodiment, a plurality of light receiving elements may be a region of one surface of the housing of an electronic device (e.g., the electronic device 101 of FIG. 2) surrounding at least part of the light emitting unit 210.

According to an embodiment, each of the first light receiving unit 221, the second light receiving unit 222, the third light receiving unit 223, and the fourth light receiving unit 224 may be configured to receive the light of a specified wavelength via multiple channels in which a single band is spatially separated. For example, each of the first light receiving unit 221, the second light receiving unit 222, the third light receiving unit 223, and the fourth light receiving unit 224 may be configured to detect light of a band including all the plurality of LEDs 211, 212, 213, and 214 of the light emitting unit 210. According to an embodiment, at least one of the first light receiving unit 221, the second light receiving unit 222, the third light receiving unit 223, and the fourth light receiving unit 224 may be configured to detect light of a band different from that of another light receiving unit. For example, the first light receiving unit 221 may be configured to detect light of a first band, and the second light receiving unit 222 may be configured to detect light of a second band different from the first band.

According to various embodiments, biometric information may be measured based on the distance of the light emitting element to each light receiving element.

Referring to FIG. 4, for example, the first LED 211 and the second LED 212 may be positioned at a distance spaced from the first light receiving unit 221 by a first distance D1. The third LED 213 and the fourth LED 214 may be positioned at a distance spaced from the first light receiving unit 221 by a second distance D2. According to an embodiment, at least a piece of biometric information may be measured based on the difference between the first distance D1 and the second distance D2. According to an embodiment, the light receiving unit may be determined to measure biometric information depending on a wavelength of the light of a light emitting unit for the distance difference-based biometric information measurement. For example, the first LED 211 and the third LED 213 may be used to measure first biometric information. Because the third LED 213 emits light of a wavelength longer than the first LED 211, the first light receiving unit 221, which is positioned at the first distance D1 from the first LED 211 and which is positioned at the second distance D2 from the third LED 213, may be used. For another example, the second LED 212 and the third LED 213 may be used to measure second biometric information. Because the third LED 213 emits light of a wavelength longer than the second LED 212, the first light receiving unit 221 and/or the fourth light receiving unit 224, which is positioned at the first distance D1 from the second LED 212 and which is positioned at the second distance D2 from the third LED 213, may be used to measure the second biometric information. According to an embodiment, a light receiving unit positioned at the same distance from a plurality of light emitting units may be used to measure biometric information. For example, the third LED 213 and the fourth LED 214 may be used to measure third biometric information. In this case, the first light receiving unit 221 positioned at the second distance (e.g., D2) from the third LED 213 and the fourth LED 214 and/or the third light receiving unit 223 positioned at the first distance (e.g., D1) from the third LED 213 and the fourth LED 214 may be used to measure the third biometric information.

According to various embodiments, an electronic device (e.g., the electronic device 101 of FIG. 2) may measure biometric information, using a plurality of light emitting elements. According to an embodiment, the electronic device 101 may detect at least one light having a specified wavelength, using a plurality of light emitting elements and may obtain biometric information, using at least part of light detected from the plurality of light emitting elements. For example, the electronic device 101 may obtain the biometric information based on the sum or average of optical signals detected from a plurality of light emitting elements. According to an embodiment, the electronic device 101 may obtain the biometric information, using light having the quality (e.g., a signal-to-noise ratio (SNR) of a specified range or more) of a specified range or more, in the light detected from a plurality of light emitting elements. For example, the electronic device 101 may obtain the biometric information, based on the sum or average of optical signals having the quality of a specified range or more.

According to various embodiments, the electronic device 101 may use a plurality of light receiving units with respect to one light to improve SNR. For example, the plurality of light receiving units of the biometric information sensor 201 may experience different noise environments, based on the wearing environment of the electronic device 101. According to an embodiment, the electronic device 101 may estimate information about noise (e.g., optical noise from the outside) using the plurality of light receiving units. For example, when the first LED 211 is used to measure the biometric information, the electronic device 101 may estimate noise information based on the difference in light measured by the first light receiving unit 221 and the second light receiving unit 222 spaced from the first LED 211 by the first distance D1 and/or the difference in light measured by the third light receiving unit 223 and the fourth light receiving unit 224 spaced from the first LED 211 by the second distance D2. For example, when the second LED 212 is used to measure the biometric information, the electronic device 101 may estimate noise information based on the difference in light measured by the first light receiving unit 221 and the fourth light receiving unit 224 spaced from the second LED 212 by the first distance D1 and/or the difference in light measured by the second light receiving unit 222 and the third light receiving unit 223 spaced from the second LED 212 by the second distance D2. For example, when the third LED 213 is used to measure the biometric information, the electronic device 101 may estimate noise information based on the difference in light measured by the second light receiving unit 222 and the third light receiving unit 223 spaced from the third LED 213 by the first distance D1 and/or the difference in light measured by the first light receiving unit 221 and the fourth light receiving unit 224 spaced from the third LED 213 by the second distance D2. For example, when the fourth LED 214 is used to measure the biometric information, the electronic device 101 may estimate noise information based on the difference in light measured by the third light receiving unit 223 and the fourth light receiving unit 224 spaced from the fourth LED 214 by the first distance D1 and/or the difference in light measured by the first light receiving unit 221 and the second light receiving unit 222 spaced from the fourth LED 214 by the second distance D2. According to an embodiment, the electronic device 101 may improve the SNR of the biometric information measured using the estimated noise information. For example, the electronic device 101 may improve the SNR of the biometric information using a method such as interference or noise cancellation. For another example, the electronic device 101 may improve the SNR by adjusting the light intensity of at least one LED of the light emitting unit 210 based on the estimated noise information. The electronic device 101 may improve the SNR by increasing the light intensity of at least one LED of the light emitting unit 210, when the estimated noise information is above the specified range.

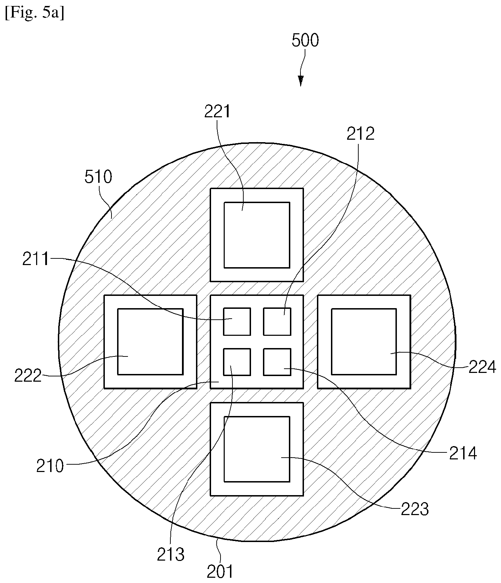

FIG. 5a illustrates a first example of a biometric information sensor, according to various embodiments of the disclosure.

Referring to FIG. 5a, according to various embodiments, in a first example 500, the biometric information sensor 201 may include a plurality of light receiving units (e.g., a light receiving element, a photodetector, and/or a photo diode) arranged around the light emitting unit 210. According to an embodiment, the biometric information sensor 201 may include the first light receiving unit 221, the second light receiving unit 222, the third light receiving unit 223, and the fourth light receiving unit 224. For example, the plurality of light receiving units 221, 222, 223, and 224 may be located on different sides of the light emitting unit 210, respectively.

According to various embodiments, the biometric information sensor 201 may sense the oxygen saturation of an external object, using a light receiving unit positioned at the same distance from an infrared LED (e.g., the third LED 213) and a red LED (e.g., the fourth LED 214). For example, an electronic device (e.g., the electronic device 101 of FIG. 2) may detect oxygen saturation by detecting light emitted from the infrared LED (e.g., the third LED 213) and the red LED (e.g., the fourth LED 214) using the first light receiving unit 221 and/or the third light receiving unit 223. According to an embodiment, the electronic device 101 may emit an infrared LED (e.g., the third LED 213) and a red LED (e.g., the fourth LED 214) sequentially or simultaneously. After emitting the infrared LED (e.g., the third LED 213), the electronic device 101 may emit the red LED (e.g., the fourth LED 214). The electronic device 101 may detect the light having wavelengths corresponding to red light and infrared light, using a plurality of light receiving units. For example, the electronic device 101 may detect red light and infrared light, using the plurality of light receiving units 221, 222, 223, and 224 and may obtain oxygen saturation, using the light detected by the first light receiving unit 221 and/or the third light receiving unit 223. For another example, the electronic device 101 may detect red light and infrared light, using the first light receiving unit 221 and/or the third light receiving unit 223 among the plurality of light receiving units and may obtain the oxygen saturation, using the detected light. For example, the electronic device 101 may activate only the first light receiving unit 221 and/or the third light receiving unit 223. According to an embodiment, the electronic device 101 may obtain the oxygen saturation, based on at least part of the light obtained from the plurality of light receiving units. For example, the electronic device 101 may obtain the oxygen saturation, using an optical signal, which has a good quality, from among optical signals obtained by the first light receiving unit 221 or the third light receiving unit 223. For example, the electronic device 101 may obtain the oxygen saturation, based on the average or sum of optical signals obtained by the first light receiving unit 221 and the third light receiving unit 223.

According to various embodiments, the electronic device 101 may measure blood glucose of an external object, using a light receiving unit positioned at different distances from each of a blue LED (e.g., the second LED 212) and an infrared LED (e.g., the third LED 213) of the biometric information sensor 201. According to an embodiment, the electronic device 101 may measure the blood glucose, using a light receiving unit, which is relatively close to the blue LED (e.g., the second LED 212) and which is relatively far from the infrared LED (e.g., the third LED 213). For example, the biometric information sensor 201 may sense the blood glucose, using the first light receiving unit 221 and/or the fourth light receiving unit 224. According to an embodiment, the electronic device 101 may emit an infrared LED (e.g., the third LED 213) and a blue LED (e.g., the second LED 212) sequentially or simultaneously. After emitting the infrared LED (e.g., the third LED 213), the electronic device 101 may emit the blue LED (e.g., the second LED 212). The electronic device 101 may detect the light having wavelengths corresponding to blue light and infrared light, using a plurality of light receiving units. For example, the electronic device 101 may detect blue light and infrared light, using the plurality of light receiving units 221, 222, 223, and 224 and may obtain blood glucose information, using the light detected by the first light receiving unit 221 and/or the fourth light receiving unit 224. For another example, the electronic device 101 may detect blue light and infrared light, using the first light receiving unit 221 and/or the fourth light receiving unit 224 among the plurality of light receiving units and may obtain the blood glucose information, using the detected light. For example, the electronic device 101 may activate only the first light receiving unit 221 and/or the fourth light receiving unit 224. According to an embodiment, the electronic device 101 may obtain the blood glucose information, based on at least part of the light obtained from the plurality of light receiving units. For example, the electronic device 101 may obtain the blood glucose information, using an optical signal, which has a good quality (e.g., SNR), from among optical signals obtained by the first light receiving unit 221 or the fourth light receiving unit 224. For example, the electronic device 101 may obtain the blood glucose information, based on the average or sum of optical signals obtained by the first light receiving unit 221 and the fourth light receiving unit 224.

According to various embodiments, the electronic device 101 may detect heart rate information or blood pressure information, using all of the four light receiving units 221, 222, 223, and 224. For example, the electronic device 101 may obtain accurate heart rate information or blood pressure information, by using a plurality of light receiving units. According to an embodiment, the electronic device 101 may obtain blood pressure information of an external object, using the plurality of light receiving units 221, 222, 223, and 224. The electronic device 101 may emit a green LED (e.g., the first LED 211) and may detect green light, using the plurality of light receiving units 221, 222, 223, and 224. For example, the electronic device 101 may obtain the blood pressure information, by accumulating and/or comparing the data of the optical signals detected by the plurality of light receiving units. The electronic device 101 may obtain the blood pressure information, based on the average or sum of the optical signals detected by the plurality of light receiving units 221, 222, 223, and 224. The electronic device 101 may obtain the blood pressure information, using only the optical signal having the quality (e.g., SNR of a specified range or more) of a specified range or more, from among the optical signals detected by the plurality of light receiving units. According to an embodiment, the electronic device 101 may obtain the heart rate information of an external object, using the plurality of light receiving units. The electronic device 101 may emit a green LED (e.g., the first LED 211) and an infrared LED (e.g., the third LED 213) simultaneously or sequentially and may detect green light and infrared light, using the plurality of light receiving units. For example, the electronic device 101 may obtain the heart rate information, by accumulating and/or comparing the data of the optical signals detected by the plurality of light receiving units. For example, the electronic device 101 may obtain the heart rate information, based on the average or sum of the optical signals detected by the plurality of light receiving units. The electronic device 101 may obtain the heart rate information, using only the optical signal having the quality (e.g., SNR of a specified range or more) of a specified range or more, from among the optical signals detected by the plurality of light receiving units.

According to various embodiments, the biometric information sensor 201 may include a partition structure 510. According to an embodiment, the partition structure 510 may be formed to reduce crosstalk due to the plurality of LEDs 211, 212, 213, and 214. For example, the partition structure 510 may include a partition positioned between the light emitting unit 210 and the plurality of light receiving units 221, 222, 223, and 224.

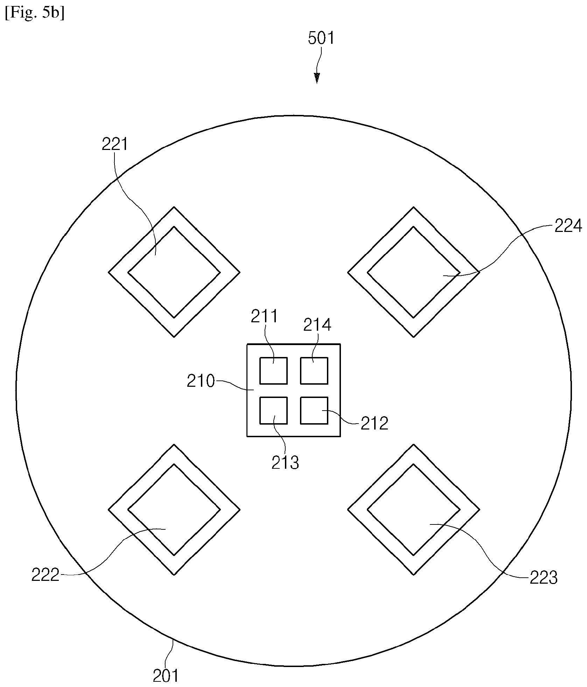

FIG. 5b illustrates a second example of a biometric information sensor, according to various embodiments of the disclosure.

Referring to FIG. 5b, according to various embodiments, in a second example 501, the biometric information sensor 201 may include the plurality of light receiving units 221, 222, 223, and/or 224 positioned adjacent to each vertex of the light emitting unit 210.

According to various embodiments, the biometric information sensor 201 may sense the oxygen saturation of an external object, using an infrared LED (e.g., the third LED 213) and a red LED (e.g., the fourth LED 214). According to an embodiment, an electronic device (e.g., the electronic device 101 of FIG. 2) may emit the infrared LED (e.g., the third LED 213) and the red LED (e.g., the fourth LED 214) simultaneously or sequentially and may detect red light or infrared light, using at least one light receiving unit which is positioned at the same distance from the infrared LED (e.g., the third LED 213) and the red LED (e.g., the fourth LED 214). For example, the electronic device 101 may detect red light and infrared light, using the first light receiving unit 221 and/or the third light receiving unit 223. For another example, the electronic device 101 may detect infrared light, using the first light receiving unit 221 and may detect the red light, using the fourth light receiving unit 224.

According to various embodiments, the electronic device 101 may measure blood glucose of an external object, using a light receiving unit positioned at different distances from each of a blue LED (e.g., the second LED 212) and an infrared LED (e.g., the third LED 213) of the biometric information sensor 201. According to an embodiment, the electronic device 101 may measure the blood glucose, using a light receiving unit, which is relatively close to the blue LED (e.g., the second LED 212) and which is relatively far from the infrared LED (e.g., the third LED 213). For example, the biometric information sensor 201 may measure blood glucose, by detecting blue light and infrared light using the third light receiving unit 223 and/or the fourth light receiving unit 224.

According to various embodiments, the electronic device 101 may detect heart rate information, using all of the four light receiving units 221, 222, 223, and 224. For example, the electronic device 101 may obtain accurate heart rate information or blood pressure information, by using a plurality of light receiving units. According to an embodiment, the electronic device 101 may obtain the blood pressure information of an external object, using the plurality of light receiving units. The electronic device 101 may emit a green LED (e.g., the first LED 211) and may detect green light, using the plurality of light receiving units. For example, the electronic device 101 may obtain the blood pressure information, by accumulating and/or comparing the data of the optical signals detected by the plurality of light receiving units. For example, the electronic device 101 may obtain the blood pressure information, based on the average or sum of the optical signals detected by the plurality of light receiving units. The electronic device 101 may obtain the blood pressure information, using only the optical signal having the quality (e.g., SNR of a specified range or more) of a specified range or more, from among the optical signals detected by the plurality of light receiving units. According to an embodiment, the electronic device 101 may obtain the heart rate information of an external object, using the plurality of light receiving units. The electronic device 101 may emit a green LED (e.g., the first LED 211) and an infrared LED (e.g., the third LED 213) simultaneously or sequentially and may detect green light and infrared light, using the plurality of light receiving units. For example, the electronic device 101 may obtain the heart rate information, by accumulating and/or comparing the data of the optical signals detected by the plurality of light receiving units. For example, the electronic device 101 may obtain the heart rate information, based on the average or sum of the optical signals detected by the plurality of light receiving units. The electronic device 101 may obtain the heart rate information, using only the optical signal having the quality (e.g., SNR of a specified range or more) of a specified range or more, from among the optical signals detected by the plurality of light receiving units.

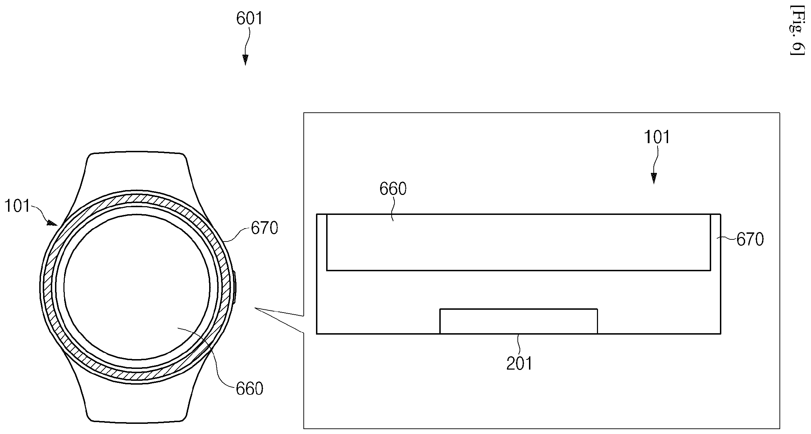

FIG. 6 illustrates a configuration of an electronic device, according to various embodiments of the disclosure.

Referring to FIG. 6, according to various embodiments, in a configuration 601, an electronic device (e.g., the electronic device 101 of FIG. 2) may be a wearable device. According to an embodiment, the electronic device 101 may include a display 660 (e.g., the display device 160 of FIG. 1) positioned on the front side of housing 670. According to an embodiment, the electronic device 101 may include the biometric information sensor 201 positioned on the rear side of the housing 670. For example, the biometric information sensor 201 may sense biometric information of a wearer, via at least part of the housing 670. For example, the biometric information sensor 201 may be implemented in the form described above with regard to FIGS. 4, 5a, and 5b.

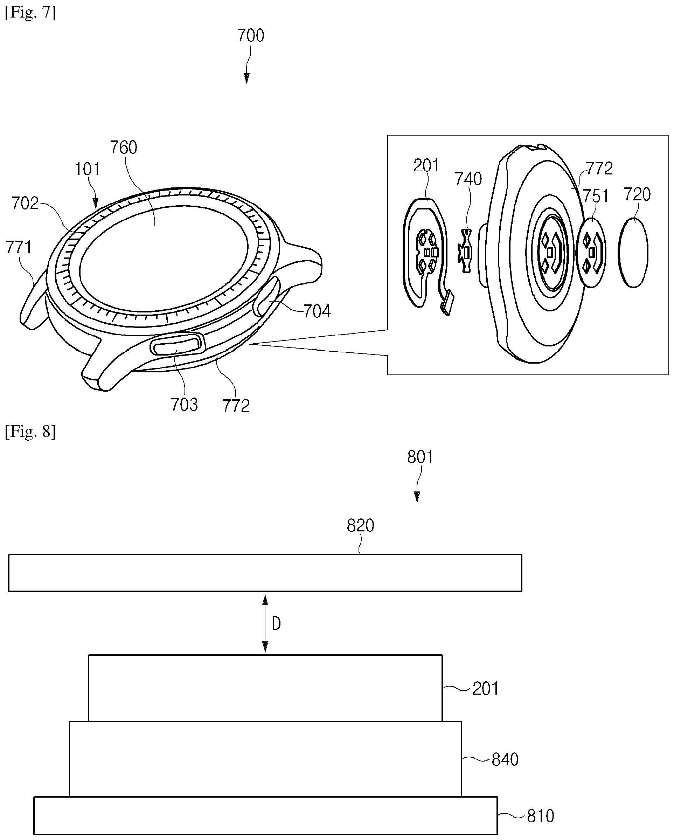

FIG. 7 illustrates a mounting structure of a biometric information sensor of an electronic device, according to various embodiments of the disclosure.

Referring to FIG. 7, according to various embodiments, a mounting structure 700 of an electronic device (e.g., the electronic device 101 of FIG. 2) may include a front housing 771 and a rear housing 772. For example, the front housing 771 and the rear housing 772 may correspond to the housing 670 of FIG. 6. According to an embodiment, a display 760 (e.g., the display device 160 of FIG. 2) may be exposed through one surface of the front housing 771. Key input devices 702, 703, and 704 may be positioned in at least part of the front housing 771.

According to an embodiment, the biometric information sensor (e.g., the biometric information sensor 201 of FIG. 2) may be positioned on one surface of the rear housing 772. For example, a first structure 740 may be positioned between the biometric information sensor 201 and the rear housing 772. The first structure 740 may be used to fix the biometric information sensor 201 on the rear housing 772. The first structure 740 may be used to adjust the interval between the biometric information sensor 201 and a cover glass 720.

According to an embodiment, the biometric information sensor 201 may be configured to obtain biometric information by emitting light via the cover glass 720 positioned on one surface of the rear housing 772 and receiving the reflected light. For example, the cover glass 720 may be formed of a transparent material.

According to an embodiment, a second structure 751 may be positioned between the cover glass 720 and the rear housing 772. For example, the second structure 751 may be used to protect the elements of the biometric information sensor 201. The second structure 751 may be formed of a light-absorbing material or may have a light-absorbing color. The second structure 751 may be used to reduce noise due to light reflection. The second structure 751 may be used as a partition structure. For example, the second structure 751 may be used to reduce crosstalk.

FIG. 8 illustrates a configuration on a printed circuit board (PCB) of a biometric information sensor, according to various embodiments of the disclosure.

Referring to FIG. 8, according to various embodiments, in a configuration 800, an electronic device (e.g., the electronic device 101 of FIG. 2) may include a window 820 (e.g., the cover glass 720 of FIG. 7) formed in at least part of housing (e.g., the rear housing 772 of FIG. 7). For example, the window 820 may be formed of a transparent material and may protect the biometric information sensor 201.

According to various embodiments, the biometric information sensor 201 may be mounted on the PCB 810 of the electronic device 101 together with an interposer 840. According to an embodiment, the interposer 840 may be implemented in a structure for electrically and/or physically connecting an electrical circuit on the PCB 810 to the biometric information sensor 201. According to an embodiment, the interposer 840 may be positioned to adjust a distance D between the biometric information sensor 201 and the window 820.

An embodiment is exemplified in FIGS. 6 to 8 as the biometric information sensor 201 is positioned on the rear side of the electronic device 101. However, the location of the biometric information sensor 201 is not limited thereto. For example, as described later, the biometric information sensor 201 may be positioned on the front side of the electronic device 101. For example, the biometric information sensor 201 may be implemented in an in-display form.

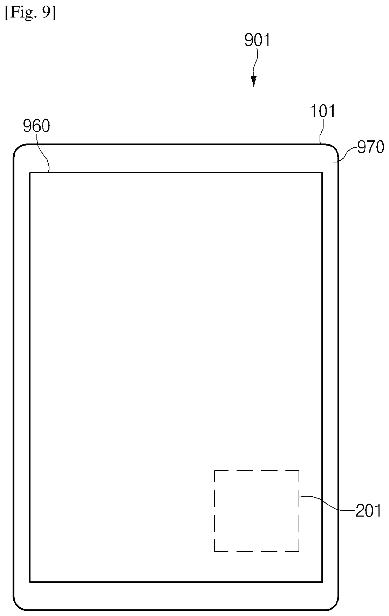

FIG. 9 illustrates another configuration of an electronic device, according to various embodiments of the disclosure.

Referring to FIG. 9, according to various embodiments, an electronic device (e.g., the electronic device 101 of FIG. 2) may be a mobile phone. Referring to an exemplification 901 of FIG. 9, the electronic device 101 may include a display 960 (e.g., the display device 160 of FIG. 2) positioned on the front side of housing 970.

According to various embodiments, the electronic device 101 may include a biometric information sensor (e.g., the biometric information sensor 201 of FIG. 2) formed in a partial region of the display 960. According to an embodiment, the biometric information sensor 201 may be mounted in a display 960 in an in-display form. The location and shape of the biometric information sensor 201 illustrated in FIG. 9 is an aspect and the location and form of the biometric information sensor 201 of the disclosure is not limited thereto. The shape of the electronic device 101 of FIG. 9 is an aspect, and the biometric information sensor 201 may be implemented on the display (e.g., the display 660 of FIG. 6) of the electronic device 101 of FIG. 6 in an in-display form.

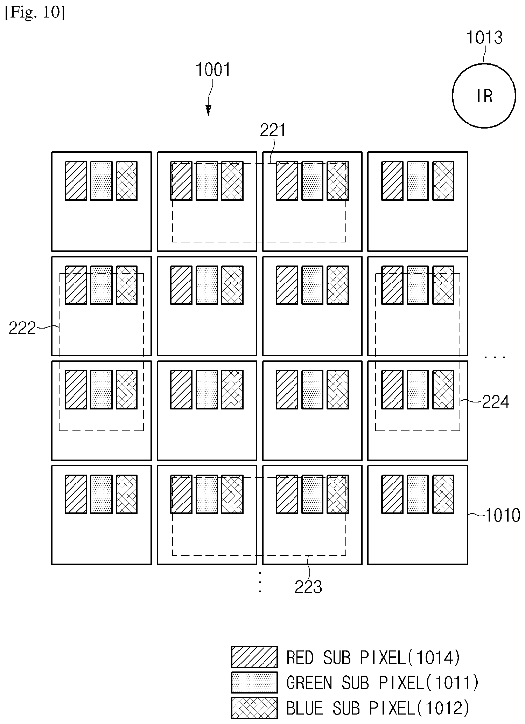

FIG. 10 illustrates a biometric information sensor in an in-display form, according to various embodiments of the disclosure.

As described above with reference to FIG. 9, according to various embodiments, a biometric information sensor (e.g., the biometric information sensor 201 of FIG. 2) may be implemented in an in-display form.

Referring to FIG. 10, according to an embodiment, the display (e.g., the display device 160 of FIG. 2) of an electronic device (e.g., the electronic device 101 of FIG. 2) may include a plurality of pixels 1010. The one pixel 1010 may include a red sub pixel 1014, a green sub pixel 1011, and a blue sub pixel 1012. In an embodiment 1001 of FIG. 10, an infrared LED 1013 may be positioned in an external region of a display or in a partial region of the display.

According to an embodiment, the electronic device 101 may include the plurality of light receiving units 221, 222, 223, and 224 in the partial region of the display. For example, the plurality of light receiving units 221, 222, 223, and 224 may be positioned on a layer below a layer on which the pixel 1010 is positioned.

According to an embodiment, the electronic device 101 may emit at least one light having a specified wavelength, using at least one sub pixel and/or an infrared LED 1013 of a display and may be configured to detect light having a specified wavelength using at least one light receiving unit among the plurality of light receiving units 221, 222, 223, and 224. For example, the electronic device 101 may obtain biometric information, using the detected light.

The structure of the pixel 1010 illustrated in FIG. 10 is an aspect, and the structure of the pixel 1010 of the disclosure is not limited thereto.

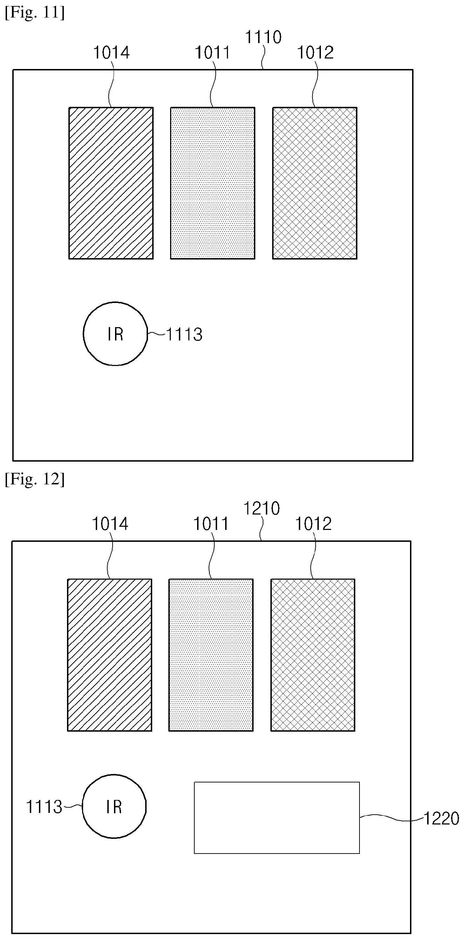

FIG. 11 illustrates an exemplification of a structure of a pixel, according to various embodiments of the disclosure.

Referring to FIG. 11, according to an embodiment, a pixel 1110 may include the green sub pixel 1011, the blue sub pixel 1012, the infrared LED 1113, and the red sub pixel 1014. For example, unlike the fact that the infrared LED 1013 of FIG. 10 is located outside the pixel 1010, referring to FIG. 11, the infrared LED 1113 may be located inside the pixel 1110. For example, the infrared LED 1113 may be implemented as a sub pixel of the pixel 1110. For example, at least part of the plurality of pixels 1010 illustrated in FIG. 10 may be substituted with the pixel 1110 of FIG. 11, instead of the infrared LED 1013 of FIG. 10.

FIG. 12 illustrates another exemplification of a structure of a pixel, according to various embodiments of the disclosure.

Referring to FIG. 12, according to an embodiment, a pixel 1210 may include the green sub pixel 1011, the blue sub pixel 1012, the infrared LED 1113, the red sub pixel 1014, and a photodetector 1220. According to an embodiment, the photodetector 1220 positioned in the pixel 1210 of FIG. 12 may be used instead of the plurality of light receiving units 221, 222, 223, and 224 of FIG. 10. For example, the photodetector 1220 may be mounted as a sub pixel of the pixel 1210. For example, the infrared LED 1013 of FIG. 10 and at least part of the plurality of light receiving units 221, 222, 223, and 224 illustrated in FIG. 10 may be substituted with the pixel 1210 of FIG. 12.

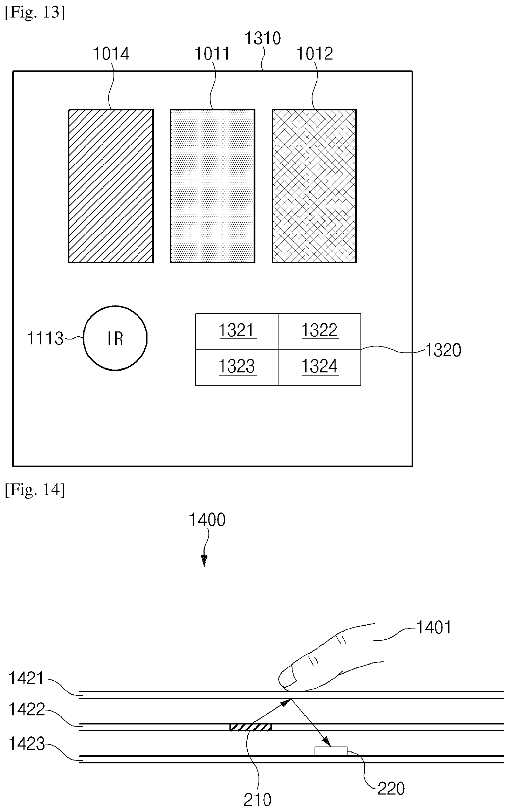

FIG. 13 illustrates still another exemplification of a structure of a pixel, according to various embodiments of the disclosure.

Referring to FIG. 13, a pixel 1310 may include the green sub pixel 1011, the blue sub pixel 1012, the infrared LED 1113, the red sub pixel 1014, and a photodetector 1320. According to an embodiment, the photodetector 1320 may include a plurality of photodetectors 1321, 1322, 1323, and 1324. The plurality of photodetectors 1321, 1322, 1323, and 1324 may be configured to detect light of different wavelengths. For example, the first photodetector 1321 may be configured to detect red light; the second photodetector 1322 may be configured to detect green light; the third photodetector 1323 may be configured to detect infrared light; the fourth photodetector 1324 may be configured to detect blue light.

FIG. 14 illustrates a layer structure for sensing biometric information, according to various embodiments of the disclosure.

Referring to FIG. 11, according to an embodiment, in a layer structure 1400, an electronic device (e.g., the electronic device 101 of FIG. 2) may obtain biometric information via a display (e.g., the display device 160). For example, the light emitting unit 210 (e.g., the light emitting unit 210 of FIG. 2) may be positioned on a display layer 1422. For example, at least one pixel (e.g., the pixel 1010 of FIG. 10, the pixel 1110 of FIG. 11, the pixel 1210 of FIG. 12, or the pixel 1310 of FIG. 13) may operate as the light emitting unit 210. The light emitted from the light emitting unit 210 may be reflected by an external object 1401 (e.g., a user) positioned on a cover 1421 (e.g., cover glass).

According to an embodiment, the electronic device 101 may detect light reflected from the external object 1401 (e.g., a user) after the light is emitted from the light emitting unit 210, using the light receiving unit 220 (e.g., the light receiving unit 220 of FIG. 2). According to an embodiment, the light receiving unit 220 may be positioned on a third layer 1423 located below the display layer 1422.

An embodiment is exemplified in FIG. 14 as the light receiving unit 220 is positioned on the third layer 1423. However, the light receiving unit 220 may be positioned on the display layer 1422.

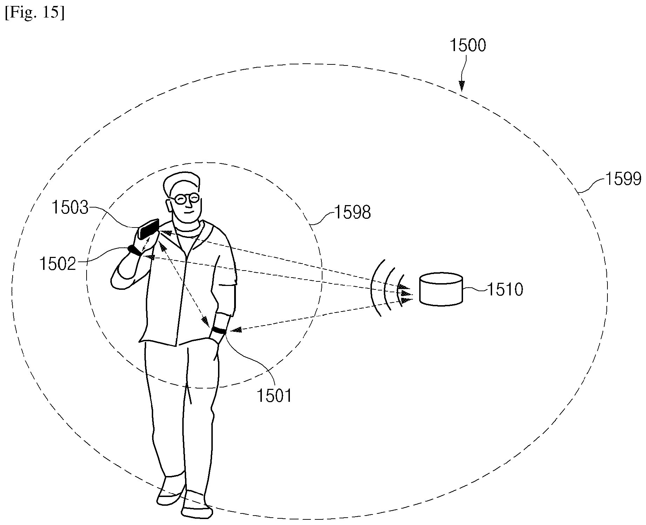

FIG. 15 illustrates a biometric information management environment, according to various embodiments of the disclosure.

Referring to FIG. 15, in a biometric information management environment 1500, each of a first electronic device 1501, a second electronic device 1502, and a third electronic device 1503 may have a structure similar to that of the electronic device 101 of FIG. 2. For example, each of the first electronic device 1501, the second electronic device 1502, and the third electronic device 1503 may include a biometric information sensor (e.g., the biometric information sensor 201 of FIG. 2). According to an embodiment, the first electronic device 1501, the second electronic device 1502, and the third electronic device 1503 may communicate with one another via a first network 1598. For example, the first network 1598 may be a network based on Bluetooth, Wi-Fi, Bluetooth low energy, near field communication (NFC), or neighbor awareness networking (NAN). According to an embodiment, the first electronic device 1501, the second electronic device 1502, and/or the third electronic device 1503 may communicate with a server 1510 over a second network 1599. For example, the second network 1599 may include a cellular network, a Wi-Fi network, and/or an Internet protocol-based communication network. According to an embodiment, the server 1510 may include a database associated with biometric information about a user. For example, the server 1510 may manage biometric information received from the first electronic device 1501, the second electronic device 1502, and/or the third electronic device 1503, based on user identification information (e.g., account and/or ID).

According to various embodiments, the biometric information measured by the first electronic device 1501 and/or the second electronic device 1502 may be transmitted to the third electronic device 1503. For example, the first electronic device 1501 and/or the second electronic device 1502 may measure biometric information and/or may transmit the measured biometric information, based on a user input (e.g., a user input to the first electronic device 1501, the second electronic device 1502, and/or the third electronic device 1503). For another example, the first electronic device 1501 and/or the second electronic device 1502 may measure biometric information and/or may transmit the measured biometric information, in response to a specified event (e.g., a specified period, turn-on, or pairing with the third electronic device 1503). According to an embodiment, the first electronic device 1501 and/or the second electronic device 1502 may be paired with the third electronic device 1503 based on the first protocol of the first network 1598.

According to various embodiments, the biometric information measured by the first electronic device 1501 and/or the second electronic device 1502 may be transmitted to the server 1510. According to an embodiment, the biometric information measured by the first electronic device 1501 and/or the second electronic device 1502 may be directly transmitted to the server 1510. For example, the first electronic device 1501 and/or the second electronic device 1502 may include a communication circuit for wireless communication with the server 1510. According to an embodiment, the biometric information measured by the first electronic device 1501 and/or the second electronic device 1502 may be directly transmitted to the server 1510 via the third electronic device 1503. For example, the third electronic device 1503 may transmit the obtained or accumulated biometric information to the server 1510 via the second network 1599. According to an embodiment, the server 1510 may update the database of the related user, based on the received biometric information.

According to various embodiments, an electronic device (e.g., the electronic device 101 of FIG. 2) may include housing (the housing 670 of FIG. 6). For example, a first surface (e.g., the rear side of the housing) of the housing may include a first region (e.g., the light emitting unit 210 of FIG. 5a) and a second region (e.g., the region of the first surface of the housing other than the light emitting unit 210 of FIG. 5a) at least partly surrounding the first region. LEDs (e.g., the first LED 211, the second LED 212, the third LED 213, and/or the fourth LED 214 of FIG. 5a) of a first number may be positioned in the first region. The first number may be an integer greater than or equal to 2, and the LEDs may be configured to emit lights having different wavelengths. Photodetectors (e.g., the first light receiving unit 221, the second light receiving unit 222, the third light receiving unit 223, and/or the fourth light receiving unit 224 of FIG. 5a) of a second number may be positioned in the second region. The second number may be an integer greater than or equal to 2. The electronic device (e.g., the electronic device 101 of FIG. 2) may include a control circuit (e.g., the signal processing unit 230 or the processor 120 of FIG. 2) operatively connected to the LEDs and the photodetectors. When executed, the control circuit may be configured to cause the LEDs of a third number to emit light sequentially or simultaneously and to cause the photodetectors of a fourth number to detect light from the LEDs of the third number. Furthermore, the third number may be 1 or 2, and the fourth number may be one of an integer that is not less than 2 and is not greater than the second number.

Favorably, the first region may generally have a form of a square. The first number may be 4, and the LEDs may be arranged in a matrix form and may be equidistant from a center of the first region.

Favorably, the second number may be 4. Each of the photodetectors may be positioned on one side of the first region, and the photodetectors may be equidistant from the center of the first region.

Favorably, the LEDs may include a first LED emitting first light having a first wavelength and a second LED emitting second light having a second wavelength different from the first wavelength. The photodetectors may include a first photodetector and a second photodetector. The control circuit may be configured to cause the first LED to emit the first light and to cause the second LED to emit the second light and to cause the first photodetector and the second photodetector to detect the first light and the second light.

Favorably, the first photodetector (e.g., the first light receiving unit 221 of FIG. 5a) may be positioned at a first distance from the first LED and the second LED, and the second photodetector (e.g., the third light receiving unit 223 of FIG. 5a) may be positioned at a second distance from the first LED and the second LED, and the first distance may be longer than the second distance. For example, the first LED (e.g., the third LED 213 of FIG. 5a) may be configured to emit infrared light, and the second LED (e.g., the fourth LED 214 of FIG. 5a) may be configured to emit red light.

Favorably, the first photodetector (e.g., the first light receiving unit 221 of FIG. 5a) and the second photodetector (e.g., the fourth light receiving unit 224 of FIG. 5a) may be positioned at a first distance from the first LED. The first photodetector (e.g., the first light receiving unit 221 of FIG. 5a) and the second photodetector (e.g., the fourth light receiving unit 224 of FIG. 5a) may be positioned at a second distance from the second LED (e.g., the second LED 212 of FIG. 5a), and the second distance may be shorter than the first distance. For example, the first LED (e.g., the third LED 213 of FIG. 5a) may be configured to emit infrared light, and the second LED (e.g., the second LED 212 of FIG. 5a) may be configured to emit blue light.

Favorably, the first LED (e.g., the third LED 213 of FIG. 5a) may be configured to emit infrared light, and the second LED (e.g., the first LED 211 of FIG. 5a) may be configured to emit green light. For example, the electronic device 101 may obtain biometric information by detecting green light and/or infrared light by using a plurality of light receiving units (e.g., the first light receiving unit 221, the second light receiving unit 222, the third light receiving unit 223, and/or the fourth light receiving unit 224 of FIG. 5a).

According to various embodiments, an electronic device (e.g., the electronic device 101 of FIG. 2) may include a biometric information sensor (e.g., the biometric information sensor 201 of FIG. 2) positioned in a first surface of the electronic device and a processor (e.g., the processor 120 of FIG. 2) operatively connected to the biometric information sensor. The biometric information sensor may include a plurality of LEDs (e.g., the light emitting unit 210 of FIG. 2) configured to emit light having different wavelengths and a plurality of photodetectors (e.g., the light receiving unit 220 of FIG. 2). The processor may be configured to emit at least one light, using at least one LED of the plurality of LEDs and to detect the emitted at least one light, using at least two photodetectors of the plurality of photodetectors. The plurality of LEDs may be positioned in a first region, and the plurality of photodetectors may be equidistant from a center of the first region.

Favorably, The processor may be configured to detect the first light, using a first photodetector positioned at a first distance from the first LED of the plurality of photodetectors and to detect the second light, using a second photodetector positioned at a second distance from the second LED of the plurality of photodetectors, and the first distance may be longer than the second distance. The processor may be configured to detect the first light, using a first photodetector positioned at a first distance from the first LED of the plurality of photodetectors and to detect the second light, using a second photodetector positioned at a second distance from the second LED of the plurality of photodetectors. The first distance may be longer than the second distance. For example, the first light may be infrared light, and the second light may be blue light.

Favorably, the processor may be configured to emit first light having a first wavelength, using a first LED of the plurality of LEDs and to emit second light having a second wavelength different from the first wavelength, using a second LED of the plurality of LEDs. For example, the processor may be configured to detect the first light and the second light, using a plurality of photodetectors positioned at the same distance from the first LED and the second LED of the plurality of photodetectors. The first light may be infrared light, and the second light may be red light.

Favorably, the processor may be configured to emit green light, using a first LED of the plurality of LEDs; and to detect the green light, using the plurality of photodetectors.

Favorably, the processor may be configured to emit green light, using a first LED of the plurality of LEDs, to emit infrared light, using a second LED of the plurality of LEDs, and to detect the green light and the infrared light, using the plurality of photodetectors.

According to various embodiments disclosed in the disclosure, accurate various pieces of biometric information may be detected.

Besides, a variety of effects directly or indirectly understood through this disclosure may be provided.

While the disclosure has been shown and described with reference to various embodiments thereof, it will be understood by those skilled in the art that various changes in form and details may be made therein without departing from the scope of the disclosure as defined by the appended claims.

The electronic device according to various embodiments may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101) working as a biometric information sensing device comprising:
a housing (670) including a first surface, the first surface including a first region and a second region at least partly surrounding the first region;
light emitting diodes, LEDs, (210, 211, 212, 213, 214) of a first number positioned in the first region, the first number being an integer greater than or equal to 2, and the LEDs (210, 211, 212, 213, 214) being configured to emit light having different wavelengths;
photodetectors (221, 222, 223, 224) of a second number positioned in the second region, the second number being an integer greater than or equal to 2; and
a control circuit (120, 230) operatively connected to the LEDs (210, 211, 212, 213, 214) and the photodetectors (221, 222, 223, 224),
wherein the control circuit (120, 230) is configured to:
cause the LEDs (210, 211, 212, 213, 214) of a third number to emit light sequentially or simultaneously, the third number being 1 or 2, and
cause the photodetectors (221, 222, 223, 224) of a fourth number to detect light from the LEDs (210, 211, 212, 213, 214) of the third number, and
wherein the fourth number is one of an integer that is not less than 2 and is not greater than the second number,
wherein the electronic device (101) comprises a display layer (1422) visually providing information to the outside of the electronic device (101), the display layer (1422) comprising multiple pixels, wherein at least one pixel of the multiple pixels operates as one of the LEDs (210, 211, 212, 213, 214),
wherein the photodetectors (221, 222, 223, 224) include a first photodetector (221) and a second photodetector (222), and
wherein the control circuit (120, 230) is further configured to:
estimate a signal-to-noise ratio, SNR, based on a difference between a first light detected by the first photodetector (221) and a second light detected by the second photodetector (222); and
increase a light intensity of at least one LED of the LEDs (210, 211, 212, 213, 214) in response to the estimated SNR being above a specified range.

2. The electronic device (101) of claim 1,
wherein the electronic device (101) comprises a layer structure (1400), the layer structure (1400) comprising a cover layer (1421), the display layer (1422), and a third layer (1423) below the display layer (1422), wherein the photodetectors (221, 222, 223, 224) are positioned on the third layer (1423).

3. The electronic device (101) of one of the preceding claims,
wherein the first region generally has a form of a square,
wherein the first number is 4, and
wherein the LEDs (210, 211, 212, 213, 214) are arranged in a matrix form and are equidistant from a center of the first region.

4. The electronic device (101) of claim 3,
wherein the second number is 4,
wherein each of the photodetectors (221, 222, 223, 224) is positioned on one side of the first region, and
wherein the photodetectors (221, 222, 223, 224) are equidistant from the center of the first region.

5. The electronic device (101) of claim 4,
wherein the LEDs (210, 211, 212, 213, 214) include:
a first LED (211) configured to emit first light having a first wavelength, and
a second LED (212) configured to emit second light having a second wavelength different from the first wavelength.

6. The electronic device (101) of claim 5,
wherein the first photodetector is positioned at a first distance from the first LED and the second LED, and
wherein the second photodetector is positioned at a second distance from the first LED and the second LED, and
wherein the first distance is longer than the second distance.

7. The electronic device (101) of claim 6,
wherein the first LED is further configured to emit infrared light, and
wherein the second LED is further configured to emit red light.

8. The electronic device (101) of claim 5,
wherein the first photodetector and the second photodetector are positioned at a first distance from the first LED,
wherein the first photodetector and the second photodetector are positioned at a second distance from the second LED, and
wherein the second distance is shorter than the first distance.

9. The electronic device (101) of claim 8,
wherein the first LED is further configured to emit infrared light, and
wherein the second LED is further configured to emit blue light.

10. The electronic device (101) of claim 5,
wherein the first LED is further configured to emit infrared light, and
wherein the second LED is further configured to emit green light.

## Patentansprüche

1. Elektronische Vorrichtung (101), die als eine biometrische Informationserfassungsvorrichtung arbeitet, die Folgendes umfasst:
ein Gehäuse (670) mit einer ersten Oberfläche, wobei die erste Oberfläche einen ersten Bereich und einen zweiten Bereich umfasst, der den ersten Bereich zumindest teilweise umgibt;
lichtemittierende Dioden, LEDs, (210, 211, 212, 213, 214) einer ersten Zahl, die in dem ersten Bereich positioniert sind, wobei die erste Zahl eine ganze Zahl größer oder gleich 2 ist, und die LEDs (210, 211, 212, 213, 214) konfiguriert sind, um Licht mit unterschiedlichen Wellenlängen zu emittieren;
Fotodetektoren (221, 222, 223, 224) einer zweiten Zahl, die in dem zweiten Bereich positioniert sind, wobei die zweite Zahl eine ganze Zahl größer oder gleich 2 ist; und
eine Steuerschaltung (120, 230), die mit den LEDs (210, 211, 212, 213, 214) und den Fotodetektoren (221, 222, 223, 224) verbunden ist,
wobei die Steuerschaltung (120, 230) konfiguriert ist, um:
die LEDs (210, 211, 212, 213, 214) einer dritten Zahl zu veranlassen, nacheinander oder gleichzeitig Licht zu emittieren, wobei die dritte Zahl 1 oder 2 ist, und
die Fotodetektoren (221, 222, 223, 224) einer vierten Zahl zu veranlassen, Licht von den LEDs (210, 211, 212, 213, 214) der dritten Zahl zu erfassen, und
wobei die vierte Zahl eine einer ganzen Zahl ist, die nicht kleiner als 2 und nicht größer als die zweite Zahl ist,
wobei die elektronische Vorrichtung (101) eine Anzeigeschicht (1422) umfasst, die visuell Informationen für die Außenseite der elektronischen Vorrichtung (101) bereitstellt, wobei die Anzeigeschicht (1422) mehrere Pixel umfasst, wobei mindestens ein Pixel der mehreren Pixel als eine der LEDs (210, 211, 212, 213, 214) arbeitet,
wobei die Fotodetektoren (221, 222, 223, 224) einen ersten Fotodetektor (221) und einen zweiten Fotodetektor (222) umfassen, und
wobei die Steuerschaltung (120, 230) ferner konfiguriert ist zum:
Schätzen eines Signal-Rausch-Verhältnisses, SNR, basierend auf einer Differenz zwischen einem ersten Licht, das von dem ersten Fotodetektor (221) erfasst wird, und einem zweiten Licht, das von dem zweiten Fotodetektor (222) erfasst wird; und
Erhöhen der Lichtintensität von mindestens einer LED der LEDs (210, 211, 212, 213, 214) als Reaktion darauf, dass der geschätzte SNR über einem bestimmten Bereich liegt.

2. Elektronische Vorrichtung (101) nach Anspruch 1,
wobei die elektronische Vorrichtung (101) eine Schichtstruktur (1400) umfasst, wobei die Schichtstruktur (1400) eine Abdeckschicht (1421), die Anzeigevorrichtung (1422) und eine dritte Schicht (1423) unterhalb der Anzeigevorrichtung (1422) umfasst, wobei die Fotodetektoren (221, 222, 223, 224) auf der dritten Schicht (1423) positioniert sind.

3. Elektronische Vorrichtung (101) nach einem der vorhergehenden Ansprüche,
wobei der erste Bereich generell die Form eines Quadrats aufweist,
wobei die erste Zahl 4 ist, und
wobei die LEDs (210, 211, 212, 213, 214) in einer Matrixform angeordnet sind und von einem Zentrum des ersten Bereichs äquidistant sind.

4. Elektronische Vorrichtung (101) nach Anspruch 3,
wobei die zweite Zahl 4 ist,
wobei jeder der Fotodetektoren (221, 222, 223, 224) auf einer Seite des ersten Bereichs positioniert ist, und
wobei die Fotodetektoren (221, 222, 223, 224) gleich weit vom Zentrum des ersten Bereichs entfernt sind.

5. Elektronische Vorrichtung (101) nach Anspruch 4,
wobei die LEDs (210, 211, 212, 213, 214) umfassen:
eine erste LED (211), die so konfiguriert ist, dass sie erstes Licht mit einer ersten Wellenlänge emittiert, und
eine zweite LED (212), die so konfiguriert ist, dass sie zweites Licht mit einer zweiten Wellenlänge emittiert, die sich von der ersten Wellenlänge unterscheidet.

6. Elektronische Vorrichtung (101) nach Anspruch 5,
wobei der erste Fotodetektor in einem ersten Abstand von der ersten LED und der zweiten LED positioniert ist, und
wobei der zweite Fotodetektor in einem zweiten Abstand von der ersten LED und der zweiten LED positioniert ist, und
wobei der erste Abstand größer als der zweite Abstand ist.

7. Elektronische Vorrichtung (101) nach Anspruch 6,
wobei die erste LED außerdem konfiguriert ist, um Infrarotlicht zu emittieren, und
wobei die zweite LED außerdem konfiguriert ist, um rotes Licht zu emittieren.

8. Elektronische Vorrichtung (101) nach Anspruch 5,
wobei der erste Fotodetektor und der zweite Fotodetektor in einem ersten Abstand von der ersten LED positioniert sind,
wobei der erste Fotodetektor und der zweite Fotodetektor in einem zweiten Abstand von der zweiten LED positioniert sind, und
wobei der zweite Abstand kürzer ist als der erste Abstand.

9. Elektronische Vorrichtung (101) nach Anspruch 8,
wobei die erste LED außerdem konfiguriert ist, um Infrarotlicht zu emittieren, und
wobei die zweite LED außerdem konfiguriert ist, um blaues Licht zu emittieren.

10. Elektronische Vorrichtung (101) nach Anspruch 5,
wobei die erste LED außerdem konfiguriert ist, um Infrarotlicht zu emittieren, und
wobei die zweite LED außerdem konfiguriert ist, um grünes Licht zu emittieren.

## Revendications

1. Dispositif électronique (101) fonctionnant comme un dispositif de détection d'informations biométriques comprenant :
un boîtier (670) comprenant une première surface, la première surface comprenant une première région et une deuxième région entourant au moins partiellement la première région ;
des diodes électroluminescentes, DEL, (210, 211, 212, 213, 214) d'un premier nombre positionnées dans la première région, le premier nombre étant un nombre entier supérieur ou égal à 2, et les DEL (210, 211, 212, 213, 214) étant configurées pour émettre de la lumière ayant différentes longueurs d'onde ;
des photodétecteurs (221, 222, 223, 224) d'un deuxième nombre positionnés dans la deuxième région, le deuxième nombre étant un nombre entier supérieur ou égal à 2 ; et
un circuit de commande (120, 230) connecté de manière opérationnelle aux DEL (210, 211, 212, 213, 214) et aux photodétecteurs (221, 222, 223, 224),
dans lequel le circuit de commande (120, 230) est configuré pour :
faire en sorte que les DEL (210, 211, 212, 213, 214) d'un troisième nombre émettent de la lumière de manière séquentielle ou simultanée, le troisième nombre étant 1 ou 2, et
faire en sorte que les photodétecteurs (221, 222, 223, 224) d'un quatrième nombre détectent la lumière provenant des DEL (210, 211, 212, 213, 214) du troisième nombre, et
dans lequel le quatrième nombre est un nombre entier supérieur ou égal à 2 et inférieur ou égal au deuxième nombre,
dans lequel le dispositif électronique (101) comprend une couche d'affichage (1422) fournissant visuellement des informations à l'extérieur du dispositif électronique (101), la couche d'affichage (1422) comprenant plusieurs pixels, au moins un pixel des multiples pixels fonctionnant comme l'une des DEL (210, 211, 212, 213, 214),
dans lequel les photodétecteurs (221, 222, 223, 224) comprennent un premier photodétecteur (221) et un deuxième photodétecteur (222), et
dans lequel le circuit de commande (120, 230) est en outre configuré pour :
estimer un rapport signal/bruit, SNR, sur la base d'une différence entre une première lumière détectée par le premier photodétecteur (221) et une deuxième lumière détectée par le deuxième photodétecteur (222) ; et
augmenter l'intensité lumineuse d'au moins une DEL des DEL (210, 211, 212, 213, 214) en réponse au fait que le rapport signal/bruit estimé est supérieur à une plage spécifiée.

2. Dispositif électronique (101) de la revendication 1,
dans lequel le dispositif électronique (101) comprend une structure de couche (1400), la structure de couche (1400) comprenant une couche de couverture (1421), la couche d'affichage (1422), et une troisième couche (1423) sous la couche d'affichage (1422), les photodétecteurs (221, 222, 223, 224) étant positionnés sur la troisième couche (1423).

3. Dispositif électronique (101) de l'une des revendications précédentes,
dans lequel la première région a généralement la forme d'un carré,
dans lequel le premier nombre est 4, et
dans lequel les DEL (210, 211, 212, 213, 214) sont disposées sous forme de matrice et sont équidistantes d'un centre de la première région.

4. Dispositif électronique (101) de la revendication 3,
dans lequel le deuxième nombre est 4,
dans lequel chacun des photodétecteurs (221, 222, 223, 224) est positionné sur un côté de la première région, et
dans lequel les photodétecteurs (221, 222, 223, 224) sont équidistants du centre de la première région.

5. Dispositif électronique (101) de la revendication 4,
dans lequel les DEL (210, 211, 212, 213, 214) comprennent :
une première DEL (211) configurée pour émettre une première lumière ayant une première longueur d'onde, et
une deuxième DEL (212) configurée pour émettre une deuxième lumière ayant une deuxième longueur d'onde différente de la première longueur d'onde.

6. Dispositif électronique (101) de la revendication 5,
dans lequel le premier photodétecteur est positionné à une première distance de la première DEL et de la deuxième DEL, et
dans lequel le deuxième photodétecteur est positionné à une deuxième distance de la première DEL et de la deuxième DEL, et
dans lequel la première distance est plus longue que la deuxième distance.

7. Dispositif électronique (101) de la revendication 6,
dans lequel la première DEL est en outre configurée pour émettre une lumière infrarouge, et
dans lequel la deuxième DEL est en outre configurée pour émettre une lumière rouge.

8. Dispositif électronique (101) de la revendication 5,
dans lequel le premier photodétecteur et le deuxième photodétecteur sont positionnés à une première distance de la première DEL,
dans lequel le premier photodétecteur et le deuxième photodétecteur sont positionnés à une deuxième distance de la deuxième DEL, et
dans lequel la deuxième distance est plus courte que la première distance.

9. Dispositif électronique (101) de la revendication 8,
dans lequel la première DEL est en outre configurée pour émettre une lumière infrarouge, et
dans lequel la deuxième DEL est en outre configurée pour émettre une lumière bleue.

10. Dispositif électronique (101) de la revendication 5,
dans lequel la première DEL est en outre configurée pour émettre une lumière infrarouge, et
dans lequel la deuxième DEL est en outre configurée pour émettre une lumière verte.
